# EUROPÄISCHE PATENTSCHRIFT

(11) **EP 1 583 779 B1**
(45) Veröffentlichungstag und Bekanntmachung des Hinweises auf die Patenterteilung: **14.06.2006**
(21) Anmeldenummer: 03785770.3
(22) Anmeldetag: 08.12.2003
(51) Int. Cl.: C08F 20/06, A61L 15/60, C07C 51/43

(54) **VERFAHREN ZUR HERSTELLUNG GERUCHSARMER HYDROGEL-BILDENDER POLYMERISATE**
METHOD FOR THE PRODUCTION OF LOW-ODOR HYDROGEL-FORMING POLYMERS
PROCEDE POUR PRODUIRE DES POLYMERES FORMANT UN HYDROGEL PEU ODORANT

(30) Priorität: 09.12.2002 DE 10257449
(43) Veröffentlichungstag der Anmeldung: 12.10.2005
(73) Patentinhaber: BASF Aktiengesellschaft, 67056 Ludwigshafen (DE)
(72) Erfinder: NESTLER, Gerhard, A-1070 Wien (AT); MÜLLER-ENGEL, Klaus Joachim, 76297 Stutensee (DE); WICKEL, Stefan, 67281 Bissersheim (DE)
(74) Vertreter: Reitstötter - Kinzebach
(86) Internationale Anmeldenummer: PCT/EP2003/013883
(87) Internationale Veröffentlichungsnummer: WO 2004/052949

(56) Entgegenhaltungen:
- EP-A- 0 372 706
- EP-A- 0 574 260

## Beschreibung

Die vorliegende Erfindung betrifft ein Verfahren zur Herstellung Hydrogel-bildender Polymerisate auf Basis von Acrylsäure.

Wasserabsorbierende Polymerisate, die auch als Hydrogel-bildende Polymerisate oder Superabsorber (Superabsorbing Polymers, im Folgenden als SAP abgekürzt) bezeichnet werden, sind in der Lage, wässrige Flüssigkeiten unter Bildung eines Hydrogels zu absorbieren und damit zu binden. SAP finden daher insbesondere in Hygieneartikeln wie Windeln, Inkontinenzeinlagen und -hosen, Damenbinden und dergleichen zur Absorption von Körperflüssigkeiten Verwendung. Einen umfassenden Überblick über SAP, ihre Anwendung und ihre Herstellung geben F.L. Buchholz und A.T. Graham (Herausgeber) in "Modern Superabsorbent Polymer Technology", Wiley-VCH, New York, 1998.

Unter den SAP sind solche auf Basis von Acrylsäure eine besonders wichtige Stoffklasse. Derartige SAP enthalten in der Regel herstellungsbedingt eine große Menge an flüchtigen bzw. eluierbaren Bestandteilen, insbesondere nicht umgesetzte Monomere (sogenannte Restmonomere) und speziell nicht umgesetzte Acrylsäure. Für die Anwendung der SAP in Hygieneartikeln, aber auch in Verpackungsmaterialien-für Lebensmittel oder als Hilfsmittel im Agrobereich werden grundsätzlich niedrige Gehalte an flüchtigen und eluierbaren Stoffen angestrebt. Auch aus ökologischer Sicht ist eine Reduzierung dieser Bestandteile wünschenswert.

Vielfältige Versuche wurden unternommen, den Gehalt flüchtiger Restmonomere in SAP auf Basis von Acrylsäure zu verringern. Eine Übersicht findet sich z. B. in EP 372706. Vorgeschlagen wurden u. A. die Bestrahlung des SAP mit ultraviolettem Licht (JP 62260906), die Zugabe von Aminen (JP-A 5040649) oder Sulfit bzw. Hydrogensulfit (US 4,306,955), die Extraktion mit hydrophilen organischen Lösungsmitteln oder mit superkritischem CO₂, die Verwendung spezieller Initiatorkombinationen, wie Redox-Initiatoren in Verbindung mit Azo-Initiatoren, oder die Verwendung von Mikroorganismen (US 4,742,114).

Aus der EP-A 372706 ist bekannt, dass Polymere auf Basis von Acrylsäure mit geringem Restmonomergehalt hergestellt werden können, wenn man eine wässrige Acrylsäure-Lösung einsetzt, die dadurch erhalten wurde, dass man zunächst eine Acrylsäure-Lösung mit einem molaren Überschuss an Base versetzt und nach einer Verweilzeit durch Zugabe weiterer Acrylsäure einen Neutralisationsgrad von 20 bis 100 % einstellt.

Aus der EP-A 574260 ist eine ähnliche Vorgehensweise bekannt, wobei man jedoch eine Acrylsäure einsetzt, die weniger als 1000 ppm ß-Hydroxypropionsäure enthält. Die Acrylsäure wird zu diesem Zweck stets frisch destilliert.

Die im Stand der Technik beschriebenen Methoden sind entweder sehr aufwendig oder verringern nur bedingt den messbaren Restmonomergehalt in SAP auf Basis von Acrylsäure. Zudem weisen diese SAP häufig einen unangenehmen Geruch auf. Dieser vermindert zwar grundsätzlich nicht ihre wasserabsorbierenden Eigenschaften, führt aber insbesondere bei Einsatz in Hygieneartikeln zu einer verringerten Kundenakzeptanz.

Der vorliegenden Erfindung liegt somit die Aufgabe zugrunde, ein Verfahren zur Herstellung von Superabsorber auf Basis von Acrylsäure bereitzustellen, mit dem SAP erhältlich sind, die einen geringen Gehalt an Restmonomeren aufweisen. Zudem sollten die Superabsorber keinen unangenehmen Geruch aufweisen.

Die Anmelderin hat nunmehr in eigenen Untersuchungen herausgefunden, dass sich der Restmonomergehalt in derartigen Superabsorbern verringern lässt, wenn man eine Acrylsäure umsetzt, die weniger als 500 ppm, bezogen auf das Gesamtgewicht an Acrylsäure, Oligomere der Acrylsäure enthält. Unter Oligomeren der Acrylsäure versteht der Fachmann Verbindungen der allgemeinen Formel I

CH₂=CH-C(O)-O-(CH₂-CH₂-C(O)-O)ₓH (I)

worin X für eine ganze Zahl von 1 bis 10 und insbesondere für 1 (Diacrylsäure) oder 2 bis 10 (Triacrylsäure und höhere Oligomere) steht. Oligomere der Acrylsäure entstehen bei der Lagerung von Acrylsäure durch einfache oder wiederholte Addition von Acrylsäure an die Doppelbindung von Acrylsäure oder an die Doppelbindung einer oligomeren Acrylsäure. Die Bildung von Oligomeren der Acrylsäure wird durch Wasser sowie durch saure oder basische Verunreinigungen in der Acrylsäure beschleunigt.

Demnach betrifft die vorliegende Erfindung ein Verfahren zur Herstellung eines geruchsarmen, Hydrogel-bildenden Polymerisats auf Basis von Acrylsäure, umfassend die folgenden Schritte:
a) Herstellung eines polymeren Hydrogels durch radikalische Polymerisation einer wenigstens 50 Gew.-% Acrylsäure enthaltenden Monomerzusammensetzung in einem wässrigen Polymerisationsmedium und Überführen des Hydrogels in ein teilchenförmiges Hydrogel oder ein Hydrogel bildendes Pulver; und gegebenenfalls
b) Behandeln des teilchenförmigen Hydrogels oder des Hydrogel bildenden Pulvers mit einer vernetzend wirkenden Substanz, die wenigstens zwei gegenüber den Carboxylgruppen des Polymerisats reaktive funktionelle Gruppen, gegebenenfalls in latenter Form, aufweist;
dadurch gekennzeichnet, dass die in Schritt a) eingesetzte Acrylsäure einen Gesamtgehalt an Oligomeren der Acrylsäure von weniger als 500 ppm, vorzugsweise nicht mehr als 400 ppm und insbesondere nicht mehr als als 300 ppm aufweist. Hier und im Folgenden sind alle ppm-Angaben Gewichtsanteile, die auf Acrylsäure bezogen sind. Es hat sich als vorteilhaft erwiesen, wenn der Gehalt an Triacrylsäure (Verbindung I mit x = 2) und höheren Oligomeren der Acrylsäure weniger als 100 ppm, insbesondere weniger als 50 ppm und speziell weniger als 10 ppm beträgt.

Acrylsäure mit einem Gesamtgehalt an Oligomeren der Acrylsäure von weniger als 500 ppm, vorzugsweise nicht mehr als 400 ppm und insbesondere nicht mehr als als 300 ppm wird vorzugsweise durch Kristallisation von Acrylsäure mit einem höheren Gehalt an diesen Verunreinigungen hergestellt. Durch Destillation der Rohacrylsäure kann ein derartiger Gehalt an Oligomeren grundsätzlich auch eingestellt werden. Geeignete Verfahren zur Kristallisation von Acrylsäure sind aus der EP-A 616998, der EP-A 648520, der EP-A 730893, der EP-A 776875, der WO 98/25889 und der WO 01/77056 bekannt. Nach den dort beschriebenen Verfahren, insbesondere nach dem in WO 01/77056 beschriebenen Verfahren, kann man ausgehend von Rohacrylsäure eine Reinacrylsäure herstellen, welche die erfindungsgemäß einzuhaltenden Maximalkonzentrationen an Oligomeren der Acrylsäure aufweist.

Für die Verwendung im erfindungsgemäßen Verfahren hat sich eine Acrylsäure bewährt, die durch eine ein- oder mehrstufige Kristallisation einer Rohacrylsäure mit einem Gesamtgehalt an Oligomeren der Acrylsäure von nicht mehr als 5 Gew.-% gewonnen wurde. Der Anteil an Oligomeren in der Rohacrylsäure liegt vorzugsweise im Bereich von 0,07 bis 3 Gew.-% und insbesondere im Bereich von 0,1 bis 2 Gew.-%. Die Rohacrylsäure kann daneben noch weitere organische Verunreinigungen enthalten, die bei der Kristallisation ebenfalls weitgehend abgetrennt werden. Der Gehalt an diesen weiteren organischen Verunreinigungen wird in der Regel weniger als 3 Gew.-% betragen. Beispiele für weitere Verunreinigungen sind aliphatische Carbonsäuren, insbesondere Essigsäure und Propionsäure, aromatische Aldehyde wie Furfural und Benzaldehyd, weiterhin Allylacrylat, Acrolein, aliphatische Aldehyde, Maleinsäure und deren Anhydrid sowie Prozessinhibitoren wie Phenothiazin (Dibenzol,4-thiazin; PTZ) und 4-Hydroxy-2,2,6,6-tetramethylpiperidin-1-oxyl (4-OH-TEMPO) oder vergleichbare Stabilisatoren, die der Acrylsäure häufig zur Stabilisierung zugesetzt werden.

Typische Rohacrylsäuren, die als Einsatzstoff für die Herstellung der erfindungsgemäß zu verwendenden Acrylsäure eingesetzt werden können, enthalten 80 bis 99,8 Gew.-%, insbesondere 98,0 bis 99,7 Gew.-% Acrylsäure, wenigstens 500 ppm, häufig 1000 ppm bis 5 Gew.-%, insbesondere 1000 ppm bis 1 Gew.-% aliphatische Carbonsäuren, speziell Essigsäure und/oder Propionsäure. Der Gehalt an aromatischen Aldehyden liegt in der Regel im Bereich von 0,005 bis 1 Gew.-%, insbesondere 0,01 bis 0,1 Gew.-% z. B. 0,005 bis 0,8 Gew.-% Furfural und 0,001 bis 0,6 Gew.-% Benzaldehyd. Der Gehalt an Prozessinhibitor, z. B. PTZ und/oder 4-OH-TEMPO beträgt in der Regel 0,005 bis 0,3 Gew.-%, insbesondere 0,02 bis 0,1 Gew.-%, jeweils bezogen auf die Bruttozusammensetzung der Rohacrylsäure. Daneben kann die zu reinigende Acrylsäure auch weitere, die Polymerisation von Acrylsäure nachteilig beeinflussende organische Verunreinigungen, z. B. Diacrylsäure oder Allylacrylat, enthalten. Der Anteil an diesen weiteren Verunreinigungen wird in der Regel 5 Gew.-%, bezogen auf die Bruttozusammensetzung der Rohacrylsäure, nicht überschreiten und liegt z. B. im Bereich von 0,001 bis 3 Gew.-%. Der Anteil an Diacrylsäure hängt naturgemäß vom Alter, d. h. der Lagerzeit, der Acrylsäure ab und kann bis zu 5 Gew.-%, häufig bis 3 Gew.-% betragen. Er liegt häufig im Bereich von 0,07 bis 3 Gew.-% und insbesondere 0,01 bis 2 Gew.-%. Der Wassergehalt in Rohacrylsäuren beträgt in der Regel nicht mehr als 5 Gew.-%, insbesondere nicht mehr als 3 Gew.-%. Es ist jedoch auch möglich Acrylsäure mit einem höheren Wassergehalt zu einzusetzen, z. B. bis 20 Gew.-%.

Geeignete Rohacrylsäuren sind bekannt und nach großtechnischen Verfahren durch katalytische Oxidation von C3-Kohlenwasserstoffen, insbesondere von Propan, Propen und deren Mischungen erhältlich, wobei die Rohacrylsäure in bekannter Weise, z. B. durch fraktionierende Kondensation, Totalkondensation, durch Absorption in ein geeignetes Absorptionsmittel, z. B. in hochsiedende organische Lösungsmittel oder in Wasser, gefolgt von einer Trennung der Acrylsäure und des Absorptionsmittels, aus dem Reaktionsgas gewonnen wird (zur Gewinnung von Rohacrylsäure via Aufnahme in ein hochsiedendes, organisches Absorptionsmittel, z. B. durch Absorption in ein Gemisch aus Diphenylether und Diphenyl siehe DE-A 21 36 396, DE-A 43 08 087 sowie Ullmanns Enzyclopedia of Ind. Chem. 5^{th} ed. on CD-ROM, loc. cit.; zur Gewinnung von Rohacrylsäure via Absorption in Wasser siehe z. B. EP-A 511 111 und dort zitierte Literatur; zur Gewinnung von Rohacrylsäure durch Totalkondensation des Reaktionsgases und anschließende Destillation mit azeotropen Schleppern siehe beispielsweise DE-A 34 29 391 und JP-A 1124766; zur Gewinnung von Rohacrylsäure durch Extraktionsverfahren mit organischen Lösungsmitteln siehe z. B. DE-A 21 64 767, JP-A 58140039, US 3,553,261, US 4,219,389, GB 1,427,223, US 3,962,074 und DE 23 23 328; zur Gewinnung von Rohacrylsäure durch fraktionierende Kondensation siehe z. B. DE-A 197 40 253 und die ältere deutsche Patentanmeldung 10053086.9). Bevorzugte Rohacrylsäure werden nach den Verfahrender EP-A 511 111 oder der älteren deutsche Patentanmeldung 10053086.9 erhalten.

Zur Durchführung der Kristallisation überführt man die Rohacrylsäure in einen Kristallisator und kristallisiert unter Kühlen einen Teil der Acrylsäure aus. Diese wird von der Mutterlauge abgetrennt und anschließend zur weiteren Verarbeitung aufgeschmolzen oder in Wasser oder wässrigem Alkali gelöst. Vorzugsweise setzt man hierbei der Acrylsäure geringe Mengen eines Stabilisators, vorzugsweise eines Hydrochinons oder eines Hydrochinonmonoalkylethers wie Hydrochinonmonomethylether zu. Die Menge liegt in der Regel im Bereich von 10 bis 500 ppm und insbesondere im Bereich von 50 bis 300 ppm.

Gegebenenfalls kann man die so erhaltene Acrylsäure einer oder mehreren, z. B. 2, 3, 4, 5 oder 6 weiteren, aufeinanderfolgenden Kristallisationsstufen zuführen, bis der gewünschte Reinheitsgrad erreicht ist. Vorzugsweise arbeitet man dabei nach dem Gegenstromprinzip, d. h. die Mutterlauge der jeweiligen Kristallisationsstufe wird der jeweils vorangehenden Kristallisationsstufe zugeführt. Gegebenenfalls führt man vor der Isolierung der Acrylsäure noch weitere Reinigungsschritte durch.

Die bei der Kristallisation anfallende acrylsäurehaltige Mutterlauge kann zur Gewinnung weiterer Acrylsäure ebenfalls ein oder mehreren, aufeinanderfolgenden, weiteren Kristallisationsstufen zugeführt werden. Vorzugsweise arbeitet man dabei nach dem Gegenstromprinzip, d. h. das aus der Mutterlauge einer vorangegangenen Kristallisationsstufe, z. B. der ersten Kristallisationsstufe, gewonnene Kristallisat wird der zu kristallisierenden Acrylsäure der vorangegangenen Kristallisationsstufe, z. B. der in der ersten Stufe zu kristallisierenden Rohacrylsäure, zugeführt.

In einer alternativen Ausführungsform wird die bei der Kristallisation anfallende Mutterlauge, bei einer mehrstufigen Kristallisation vorzugsweise die in der 1. Stufe anfallende Mutterlauge, einer einfachen Destillation oder einer fraktionierenden Destillation zugeführt. Hierbei wird die Acrylsäure über Kopf abdestilliert und die schwerflüchtigen Verunreinigungen der Mutterlauge wie Maleinsäure(anhydrid) und Prozessinhibitoren werden als Sumpf ausgeschleust. Ein Verfahren hierzu ist aus der WO 00/01657 bekannt auf die hiermit Bezug genommen wird. Zweckmäßigerweise führt man für eine einfache Destillation die Mutterlauge einem Fallfilmverdampfer zu. Die Mutterlauge kann dann einer anderen Verwendung oder der zu kristallisierenden Rohacrylsäure zugeführt werden.

Vorzugsweise führt man die Kristallisation in der jeweiligen Kristallisationsstufe so weit, dass wenigstens 20 Gew.-% und vorzugsweise wenigstens 40 Gew.-% der in der Rohacrylsäure enthaltenen Acrylsäure auskristallisiert sind. In der Regel wird man nicht mehr als 90 Gew.-%, vorzugsweise nicht mehr als 80 Gew.-% und insbesondere nicht mehr als 70 Gew.-% der in der jeweiligen Kristallisationsstufe eingesetzten Acrylsäure auskristallisieren, um eine hinreichende Reinigungswirkung zu erzielen.

Der im erfindungsgemäßen Verfahren eingesetzte Kristallisator unterliegt an sich keiner Einschränkung. Als besonders geeignet haben sich Kristallisatoren erwiesen, deren Funktion auf der Bildung von Kristallen auf gekühlten Flächen beruht. Derartige Kristallisationsverfahren werden auch als Schichtkristallisation bezeichnet. Geeignete Apparate sind in der DE-OS 17 69 123, der DE-OS 26 06 364, der EP-A 218 545, der EP-A 323 377, der CH 645278, der FR 2668946, der EP-A 616998, der EP 638520 und der US 3,597,164 beschrieben.

Zur Schichtkristallisation wird die Rohacrylsäure mit den gekühlten Flächen des Wärmetauschers in Kontakt gebracht. Dabei kühlt man die Wärmetauscherflächen des Kristallisators vorzugsweise auf Temperaturen, die bis 40 K unterhalb der Schmelztemperatur des Acrylsäure in der Rohacrylsäure liegen. Bei Erreichen des gewünschten Kristallisationsgrades wird der Abkühlvorgang beendet und die flüssige Mutterlauge abgeführt, z. B. durch Abpumpen oder Abfließen. Die Isolierung der gereinigten, kristallisierten Acrylsäure erfolgt in der Regel durch Aufschmelzen der kristallisierten Acrylsäure, z. B. durch Erwärmen der Wärmetauscherflächen auf eine Temperatur oberhalb der Schmelztemperatur der Acrylsäure und/oder durch Zufuhr einer Schmelze gereinigter Acrylsäure. Hierbei fällt die gereinigte Acrylsäure als Schmelze an und wird als solche isoliert. Auch kann man die kristalline Acrylsäure in Wasser oder wässrigem Alkali lösen und die so erhaltene Lösung gegebenenfalls nach Zusatz eines Stabilisators direkt in der nachfolgenden Polymerisation einsetzen.

Als zusätzlichen Reinigungsschritt kann man bei der Schichtkristallisation beispielsweise ein Schwitzen der auf den Wärmetauscherflächen abgeschiedenen Kristallschicht durchführen. Hierbei wird die Temperatur der Kristallschicht etwas angehoben z. B. um 0,5 bis 5 K oberhalb der Schmelztemperatur, wobei bevorzugt die höher verunreinigten Bereiche der Kristallschicht abschmelzen und so eine zusätzliche Reinigungswirkung erzielt wird. Das Schwitzprodukt wird dann der Mutterlauge zugeführt und mit dieser weiter verarbeitet. Auch kann man die Kristallschicht mit einer Reinigungsflüssigkeit, beispielsweise einer Schmelze von aufgereinigter Acrylsäure, behandeln.

Die zur Schichtkristallisation erforderliche Temperatur der Rohacrylsäure im Kristallisator hängt von ihrer Zusammensetzung ab. Die Obergrenze ist naturgemäß die Temperatur, bei der sich die bereits kristallisierte Acrylsäure mit der in der Mutterlauge enthaltenen Acrylsäure im Gleichgewicht befindet (Gleichgewichtstemperatur). Je nach Zusammensetzung des Rohprodukts liegt die Gleichgewichtstemperatur im Bereich von +5 bis +13,5 °C. Die Temperatur der zu kristallisierenden Säure liegt vorzugsweise im Bereich von 0 bis 13,5 °C und speziell im Bereich von 5 bis 12°C, wobei stark unterkühlte Schmelzen in der Regel vermieden werden. Insbesondere wird man bei der dynamischen Schichtkristallisation das Kühlmedium, das zur Abfuhr der Kristallisationswärme erforderlich ist, während des Kristallisationsvorgangs von etwa +5 bis -5 °C auf etwa -10 bis -25°C abkühlen. Bei einer statisch durchgeführten Schichtkristallisation wird man vorzugsweise das Kühlmedium während des Kristallisationsvorgangs von einer Temperatur von anfangs +5 bis -15 °C auf etwa -15 bis -30 °C gegen Ende der Kristallisation abkühlen.

In einer Ausführungsform des Kristallisationsverfahrens führt man die Schichtkristallisation in Gegenwart von Impfkristallen durch. Dabei arbeitet man vorzugsweise so, dass man vor der Kristallisation diejenigen Flächen des Kristallisators, von denen aus während der Kristallisation Kristalle wachsen, mit einer Impfschicht aus Acrylsäure belegt. Die Impfkristalle können sowohl aus der zu reinigenden Rohacrylsäure als auch aus einer Schmelze von gereinigter Acrylsäure gewonnen werden. Beispielsweise kann man Impfkristalle auf den Flächen des Kristallisators, an denen das Kristallwachstum stattfinden soll, erzeugen, indem man einen Acrylsäure-haltigen Schmelzfilm auf diesen Flächen erzeugt und diesen anfriert, beispielsweise durch Kühlen auf eine Temperatur unterhalb der Schmelztemperatur. Vorzugsweise erfolgt die Erzeugung der Impfkristalle durch Aufbringen eines Films aus einer Suspension von Acrylsäure-Kristallen in einer Acrylsäure-Schmelze und anschließendes Anfrieren dieses Films. Das Anfrieren erfolgt hier vorzugsweise bei einer Temperatur im Bereich der Gleichgewichtstemperatur. Eine derartige Suspension kann man erzeugen, indem man aus dem Rohprodukt oder einer Schmelze der gereinigten Acrylsäure eine geringe Menge an Kristallen durch Unterkühlung ausfriert. Vorzugsweise erzeugt man Impfkristalle in einer Menge von 0,1 bis 200 g/kg Schmelze und insbesondere im Bereich von 1 bis 100 g/kg Schmelze.

Die Kristallisation an Kühlflächen kann als dynamisches oder statisches Verfahren durchgeführt werden. Vorzugsweise finden dynamische Verfahren Anwendung oder Kombinationen aus statischen und dynamischen Verfahren. Dynamische Verfahren sind aus den obengenannten Druckschriften bekannt. Statische Verfahren sind beispielsweise in der US 3,597,164, der EP 323377 und der FR 2668946 beschrieben, auf die hiermit Bezug genommen wird. Bei dem statischen Verfahren findet ein Stoffaustausch in der flüssigen Phase nur durch freie Konvektion statt (ruhende Schmelze).

Bei den dynamischen Verfahren der Kristallisation wird das zu kristallisierende Rohprodukt in einer strömenden Bewegung gehalten. Dies kann durch eine erzwungene Strömung in voll durchströmten Wärmeüberträgern erfolgen, wie z. B. in der DE 2606364 beschrieben, oder durch die Aufgabe eines Rieselfilms auf eine gekühlte Wand, wie es beispielsweise in der DE-AS 1769123 und der EP-A 218545 beschrieben wird, oder mittels bewegter Kühlflächen wie Kühlwalzen oder Kühlbänder. Vorzugsweise erfolgt die dynamische Schichtkristallisation in voll durchströmten Wärmeüberträgern, beispielsweise in außen gekühlten Rohren oder Rohrbündeln.

Bei den dynamischen Schichtkristallisationsverfahren, insbesondere solchen, die in voll durchströmten Wärmeaustauschern durchgeführt werden, geht man in der Regel so vor, dass man - gegebenenfalls nach Aufbringen der Impfkristallschicht auf den Wärmetauscherflächen des Kristallisators - die Rohacrylsäure mit den gekühlten Wärmetauscherflächen in Kontakt bringt, beispielsweise indem man das Rohprodukt durch die gekühlten Rohre des Kristallisators strömen lässt. Hierbei kristallisiert Acrylsäure zumindest teilweise aus. In der Regel wird dieser Vorgang abgebrochen, wenn aufgrund der auskristallisierten Menge an Acrylsäure ein ausreichendes Durchströmen der Schmelze durch den Wärmetauscher gerade noch möglich ist. Hiernach entfernt man die flüssige Phase (Mutterlauge) und isoliert danach die kristallisierte Acrylsäure in der oben beschriebenen Weise, indem man gegebenenfalls nach einem weiteren Reinigungsschritt die Wärmetauscherflächen auf eine Temperatur oberhalb der Schmelztemperatur der Acrylsäure erwärmt. Dieser Vorgang kann mehrmals wiederholt werden, bis die gewünschte Menge an Acrylsäure aus dem Rohprodukt auskristallisiert ist.

Die Kristallisation kann als Alternative zur Schichtkristallisation auch als Suspensionskristallisation durchgeführt werden. Bei der Suspensionskristallisation wird durch Kühlen der Rohacrylsäure eine Suspension von aufgereinigten Acrylsäurekristallen in einer an Verunreinigungen angereicherten Schmelze erzeugt. Die Acrylsäurekristalle können dabei unmittelbar in der Suspension (Schmelze) wachsen oder sich als Schicht auf einer gekühlten Wand abscheiden, von der sie anschließend abgekratzt und in der Restschmelze suspendiert werden. Die Kristallsuspension wird vorzugsweise während des Suspensionskristallisationsverfahrens bewegt, wozu insbesondere ein Umpumpen oder Rühren geeignet ist. Hinsichtlich der zur Kristallisation der Acrylsäure erforderlichen Temperaturen der Schmelze gilt das oben Gesagte.

Bei der Suspensionskristallisation erfolgt die Abfuhr der Wärme in der Regel durch indirekte Kühlung, beispielsweise über Kratzkühler, die mit einem Rührkessel oder einem Behälter ohne Rührwerk verbunden sind. Der Umlauf der Kristallsuspension wird hierbei durch eine Pumpe gewährleistet. Daneben besteht auch die Möglichkeit, die Wärme über Wände des Rührkessels mit wandgängigen Rührern abzuführen. Zur Wärmeabfuhr ist auch die Verwendung von Kühlscheibenkristallisatoren geeignet, wie sie beispielsweise von der Fa. GMF (Gouda in Holland) hergestellt werden. Selbstverständlich kann man die Wärme auch durch Kühlung über herkömmliche Wärmeüberträger (bevorzugt Rohrbündel- oder Plattenwärmeüberträger) abführen. Geeignete Apparate für die Suspensionskristallisation sind z. B. in Chem.-Ing.-Techn. 57 (1985) Nr.2 S. 91-102 beschrieben.

Die Trennung des bei der Suspensionskristallisation anfallenden, mit Acrylsäure angereicherten Kristallisats von der an Acrylsäure abgereicherten Mutterlauge gelingt nach den hierzu bekannten Verfahren der Fest-Flüssig-Trennung, beispielsweise durch Filtration, Sedimentieren und/oder Zentrifugieren. Bei einem ruhenden Kristallisat kann man die Mutterlauge auch entfernen, indem man sie ablaufen lässt. Die Kristallsuspension kann auch direkt in eine Waschkolonne überführt werden, wie es in dem Verfahren der WO 01/77056 beschrieben wird, insbesondere wenn die Kristallisation der Acrylsäure im Beisein von 0,2 bis 10 Gew.-%, speziell 0,6 bis 3 Gew.-% Wasser, bezogen auf die in der Rohsäure enthaltene Acrylsäure erfolgt.

Während und/oder nach der Fest-Flüssig-Trennung können weitere Verfahrensschritte zur Steigerung der Reinheit der Kristalle bzw. des Kristallkuchens vorgesehen werden. Vorzugsweise wird man nach dem Abtrennen der Kristalle von der Mutterlauge ein ein- oder mehrstufiges Waschen und/oder Schwitzen der Kristalle oder des Kristallkuchens durchführen. Als Waschflüssigkeit wird man vorzugsweise flüssige Acrylsäure einsetzen, deren Reinheit oberhalb derer der Mutterlauge liegt. Das Waschen kann in den hierfür üblichen Apparaten erfolgen, beispielsweise in Zentrifugen oder in Filternutschen oder Bandfiltern. Das Waschen kann ein- oder mehrstufig durchgeführt werden, wobei die Waschflüssigkeit vorzugsweise im Gegenstrom zum Kristallkuchen geführt wird. Bei einer mehrstufigen Kristallisation wird als Waschflüssigkeit für das Kristallisat einer jeweiligen Kristallisationsstufe vorzugsweise der Zulauf zu derselben Kristallisationsstufe eingesetzt. Bevorzugt liegt das Massenverhältnis von Waschflüssigkeit zu Kristallisat im Bereich von 0,1 bis 1, besonders bevorzugt im Bereich von 0,2 bis 0,6 kg Waschflüssigkeit zu kg Kristallisat.

Zur Aufreinigung des bei der Suspensionskristallisation anfallenden Kristallisats werden vorzugsweise Waschkolonnen eingesetzt, in denen das Kristallisat, vorzugsweise nach einer Voreindickung z. B. durch Filtration oder Sedimentation, im Gegenstrom zu einer Waschflüssigkeit geführt wird. Vorzugsweise wird das in die Waschkolonne überführte Kristallisat nicht mehr als 30 Gew.-%, z. B. 5 bis 30 Gew.-% restliche Schmelze, bezogen auf das Kristallisat enthalten. Bei der Aufreinigung in Waschkolonnen kann man kontinuierlich oder diskontinuierlich arbeiten. Als Waschflüssigkeit wird bevorzugt eine Schmelze des bereits gereinigten Kristallisats verwendet. Der Transport der Kristalle entgegen der Strömungsrichtung kann in üblicher Weise, z. B. mittels Schwerkraft, vorzugsweise jedoch mit erzwungenem Transport der Acrylsäurekristalle, z. B. durch mechanische Förderung oder durch hydraulische Kräfte (z. B. Strömungsdruckverluste beim Durchströmen des Kristallhaufwerks) erfolgen. Geeignete Waschkolonnen sind z. B. in Chem.-Ing.-Techn. 57 (1985) Nr. 2 S. 91-102, Chem.-Ing.-Techn. 63 (1991) Nr. 9 S. 881-891, WO 99/06458, sowie in EP-A 97405, EP-A 305316, EP-A 191194, EP-A 193226, EP-A 373720, EP-A 398437, EP-A 920894, US 4735781, US 4787985, WO 00/24491 und WO 01/77056 beschrieben, auf die hiermit Bezug genommen wird. Die Temperaturdifferenz zwischen der in der Waschkolonne rückgeführten Acrylsäureschmelze und dem der Waschkolonne zugeführten Kristallisat wird häufig 2 bis 15 °C betragen und liegt insbesondere im Bereich von 2 bis 10 °C und speziell im Bereich von 2 bis 4 °C. Wegen weiterer Details hierzu sei auf den Stand der Technik insbesondere auf die WO 01/77056 verwiesen.

Alle der vorgenannten Kristallisationsverfahren können kontinuierlich oder diskontinuierlich betrieben und/oder miteinander kombiniert werden. Die bevorzugte dynamische Schichtkristallisation erfolgt vorzugsweise diskontinuierlich, insbesondere wenn sie in voll durchströmten Wärmeüberträgern, wie oben beschrieben, erfolgt. Vorzugsweise umfasst das zur Reinigung der Acrylsäure angewendete Kristallisationsverfahren wenigstens eine Schichtkristallisation.

Die bei der Kristallisation anfallende Mutterlauge kann in der vorstehend beschriebenen Weise destillativ aufgearbeitet und in die Kristallisation zurückgeführt werden.

Die nach Aufreinigung der Rohacrylsäure erhaltene Acrylsäure weist einen Gesamtgehalt an Oligomeren der Acrylsäure von weniger als 500 ppm, insbesondere nicht mehr als 400 ppm und besonders bevorzugt nicht mehr als 300 ppm auf. Dabei beträgt der Gesamtgehalt an Triacrylsäure (Verbindung I mit x = 2) und höheren Oligomeren (x = 3 bis 10) vorzugsweise weniger als 100 ppm, insbesondere weniger als 50 ppm, und speziell nicht mehr als 10 ppm, bezogen auf den Gesamtgehalt an Acrylsäure. Der Gehalt an aliphatischen Carbonsäuren wie Essigsäure und Propionsäure beträgt vorzugsweise weniger als 500 ppm, insbesondere nicht mehr als 400 ppm und besonders bevorzugt nicht mehr als 300 ppm. Ein höherer Gehalt an aliphatischen Carbonsäuren ist im Hinblick auf den Restmonomergehalt tolerabel. Ein geringer Gehalt an aliphatischen Carbonsäuren führt jedoch überraschenderweise zu geruchsarmen SAP. Vermutlich ist der unangenehme Geruch von SAP auf flüchtige Derivate dieser Säuren oder Thermolyseprodukte der Derivate zurückzuführen. Diese Derivate entstehen vermutlich bei der Herstellung des SAP durch Reaktion dieser Säuren mit z. B. Thermolyseprodukten des SAP, mit mehrwertigen Alkoholen, wie sie zur Nachvernetzung eingesetzt werden, und/oder mit sonstigen, bislang nicht bekannten Nebenprodukten der SAP-Herstellung. Die Geruchsproblematik tritt verstärkt auf, wenn die Herstellung des SAP eine Oberflächennachvernetzung umfasst, bei der man das primär hergestellte Hydrogel-bildende Acrylsäure-Polymerisat mit einer vernetzend wirkenden Substanz behandelt, die wenigstens zwei gegenüber den Carboxylgruppen des Polymerisats reaktive funktionelle Gruppen, gegebenenfalls in latenter Form, aufweist. Die Geruchsproblematik tritt insbesondere dann auf, wenn zur Herstellung der SAP eine teil- oder vollständig neutralisierte Acrylsäure eingesetzt wird.

Der Gehalt an sonstigen, von Wasser verschiedenen Verunreinigungen wie aromatischen Aldehyden, Prozessinhibitoren und sonstigen organischen Verunreinigungen, beträgt in der Regel nicht mehr als 500 ppm, insbesondere nicht mehr als 300 ppm und speziell nicht mehr als 200 ppm, wobei der Gehalt an aromatischen Aldehyden in der Regel nicht mehr als 20 ppm und speziell nicht mehr als 10 ppm beträgt. Insbesondere ist der Gehalt an von MEHQ verschiedenen Prozessinhibitoren ≤ 10 ppm. Der Gehalt an MEHQ und vergleichbaren Stabilisatoren liegt in der Regel im Bereich von 10 bis 300 ppm und insbesondere 50 bis 250 ppm.

Da sich in der Acrylsäure beim Lagern Oligomere der Acrylsäure bilden, ist es von Vorteil, die im erfindungsgemäßen Verfahren einzusetzende Acrylsäure mit dem geringen Gehalt an Oligomeren erst unmittelbar vor ihrem Einsatz in Schritt a) bereitzustellen. Mit anderen Worten, man entfernt Oligomere der Acrylsäure z. B. durch Kristallisation und/oder Destillation unmittelbar, das heißt nicht länger als 48 h insbesondere nicht länger als 24 h, vor der erfindungsgemäßen Verwendung der Acrylsäure, so dass zum Zeitpunkt ihres Einsatzes die Acrylsäure einen Oligomerengehalt von weniger als 500_ppm aufweist.

Die Herstellung des SAP auf Basis von Acrylsäure erfolgt in an sich bekannter Weise, wobei man zunächst durch radikalische Polymerisation einer wenigstens 50 Gew.-% Acrylsäure enthaltenden Monomerzusammensetzung in einem wässrigen Polymerisationsmedium ein Hydrogel herstellt. Unter wässrigem Polymerisationsmedium versteht man hier sowohl wässrige Lösungen als auch Wasser-in-Öl-Emulsionen.

Als Polymerisationsverfahren kommen insbesondere die Lösungspolymerisation, d.h. die Polymerisation in homogener wässriger Phase, und die Suspensionspolymerisation in Betracht. Einen Überblick über die zur Herstellung von Hydrogelen auf Basis von Acrylsäure eingesetzten Polymerisationsverfahren geben F.L. Buchholz und A.T. Graham (Herausgeber) in "Modern Superabsorbent Polymer Technology", S. 69 bis 117 und dort zit. Literatur.

In einer bevorzugten Ausführungsform des Verfahrens führt man die Polymerisation als Lösungspolymerisation unter Ausnutzung des Trommsdorff-Norrish-Effektes (Gelpolymerisation) durch. Zu diesem Zweck wird eine wässrige, in der Regel 10 bis 70 gew.-%ige und vorzugsweise 20 bis 60 gew.-%ige wässrige Lösung einer Acrylsäure-haltigen Monomermischung, gegebenenfalls in Gegenwart einer geeigneten Pfropfgrundlage in Gegenwart einer Radikale bildenden Substanz polymerisiert.

In dem erfindungsgemäßen Verfahren wird die Acrylsäure-haltige Monomermischung vorzugsweise in teil- oder vollständig neutralisierter Form eingesetzt, d.h. der Neutralisationsgrad aller Säuregruppen-tragenden Monomere liegt im Bereich von 20 bis 100 %, vorzugsweise im Bereich von 50 bis 100 %. Besonders bevorzugt setzt man die Monomermischung in einer wässrigen Lösung mit einem Neutralisationsgrad von 60 bis 100 % ein.

Als Neutralisationsmittel kommen Alkalimetallbasen, Ammoniak und/oder Amine in Betracht. Bevorzugt werden Alkalimetallbasen wie Natronlauge, Kalilauge, Natriumcarbonat, Natriumhydrogencarbonat, Kaliumcarbonat oder Kaliumhydrogencarbonat oder anderen Carbonaten oder Hydrogencarbonaten verwendet.

Vorzugsweise führt man die Polymerisation unter weitgehendem oder vollständigem Ausschluss von Sauerstoff durch, da Sauerstoff an sich und die in der Acrylsäure üblicherweise enthaltenen Stabilisatoren in Gegenwart von Sauerstoff die Polymerisationsreaktion stören. Vorzugsweise arbeitet man daher unter einer Inertgasatmosphäre. Als Inertgas wird insbesondere Stickstoff eingesetzt. Insbesondere hat es sich bewährt, die zu polymerisierende wässrige Monomerlösung bzw. das monomerhaltige wässrige Polymerisationsmedium vor und/oder während der Polymerisation im Schritt a) mit Inertgas zu spülen.

Die Polymerisation erfolgt in der Regel im Temperaturbereich von 0 °C bis 150 °C, vorzugsweise im Bereich von 10 °C bis 100 °C, und kann sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden.

Bezogen auf ihr Gesamtgewicht enthält die zu polymerisierende Monomermischung in der Regel:
- 50 bis 99,99 Gew.-%, vorzugsweise 70 bis 99,9 Gew.-% und insbesondere 80 bis 99,8 Gew.-% Acrylsäure als Monomer A,
- 0 bis 49,99 Gew.-%, insbesondere 0 bis 29,9 Gew.-% und insbesondere 0 bis 19,8 Gew.-% eines oder mehrerer mit Acrylsäure copolymerisierbarer, monoethylenisch ungesättigter Monomere B und
- 0,01 bis 20 Gew.-%, vorzugsweise 0,1 bis 15 Gew.-%, insbesondere 0,1 bis 5 Gew.-% und speziell 0,2 bis 3 Gew.-% wenigstens einer vernetzend wirkenden Verbindung C.

Hier und im Folgenden sind alle Gewichtsanteile auf das Gesamtgewicht aller zu polymerisierenden Monomere bezogen, wobei Gewichtsangaben von Säuregruppen-tragenden Monomeren, die auch als Salze vorliegen können, stets auf die Säureform bezogen sind.

Beispiele für geeignete Monomere B sind von Acrylsäure verschiedene Säuregruppen-tragende Monomere B1, z. B. monoethylenisch ungesättigte Mono- und Dicarbonsäuren mit vorzugsweise 4 bis 8 C-Atomen wie Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure; Halbester von monoethylenisch ungesättigten Dicarbonsäuren mit 4 bis 10 vorzugsweise 4 bis 6 C-Atomen, z. B. von Maleinsäure wie Maleinsäuremonomethylester; monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure und die Salze, insbesondere die Natrium-, Kalium- und Ammoniumsalze dieser Säuren.

Bevorzugte Monomere B1 sind Methacrylsäure, Vinylsulfonsäure, 2-Acrylamido-2-methylpropansulfonsäure oder Mischungen dieser Säuren. Der Anteil der Monomere B1 an der Gesamtmonomermenge macht, sofern erwünscht, vorzugsweise 0,1 bis 29,9 und insbesondere 0,5 bis 19,8 Gew.-%, bezogen auf die Gesamtmonomermenge aus.

Zur Optimierung von Eigenschaften der erfindungsgemäßen Polymerisate kann es sinnvoll sein, als Monomere B auch monoethylenisch ungesättigte Monomere B2 einzusetzen, die keine Säuregruppen tragen, aber mit Acrylsäure und ggf. den Monomeren B1 copolymerisierbar sind und nicht vernetzend wirken. Hierzu gehören beispielsweise monoethylenisch ungesättigte Nitrile wie Acrylnitril und Methacrylnitril, die Amide der vorgenannten monoethylenisch ungesättigten Carbonsäuren, z. B. Acrylamid, Methacrylamid, N-Vinylamide wie N-Vinylformamid, N-Vinylacetamid, N-Methylvinylacetamid, N-Vinylpyrrolidon und N-Vinylcaprolactam. Zu den Monomeren B2 zählen außerdem Vinylester gesättigter C₁-C₄-Carbonsäuren wie Vinylformiat, Vinylacetat und Vinylpropionat, Alkylvinylether mit mindestens 2 C-Atomen in der Alkylgruppe, z. B. Ethylvinylether oder Butylvinylether, Ester monoethylenisch ungesättigter C₃-C₆-Carbönsäuren, z. B. Ester aus einwertigen C₁-C₁₈-Alkoholen und Acrylsäure, Methacrylsäure oder Maleinsäure, weiterhin Acrylsäure- und Methacrylsäureester von alkoxylierten einwertigen, gesättigten Alkoholen, z. B. von Alkoholen mit 10 bis 25 C-Atomen, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen (Mₙ) der Polyalkylenglykole beispielsweise bis zu 2000 betragen können. Weiterhin geeignete Monomere B2 sind Styrol und alkylsubstituierte Styrole wie Ethylstyrol oder tert-Butylstyrol. Der Anteil der Monomere B2 an der Gesamtmonomermenge wird vorzugsweise 20 Gew.-% nicht überschreiten und macht, sofern erwünscht, vorzugsweise 0,1 bis 20 Gew.-% aus.

Als vernetzend wirkende Verbindungen C kommen solche Verbindungen in Betracht, die mindestens zwei, z. B. 2, 3, 4 oder 5 ethylenisch ungesättigte Doppelbindungen im Molekül aufweisen. Diese Verbindungen werden auch als Vernetzermonomere C1 bezeichnet. Beispiele für Verbindungen C1 sind N,N'-Methylenbisacrylamid, Polyethylenglykoldiacrylate und Polyethylenglykoldimethacrylate, die sich jeweils von Polyethylenglykolen eines Molekulargewichts von 106 bis 8500, vorzugsweise 400 bis 2000, ableiten, Trimethylolpropantriacrylat, Trimethylolpropantrimethacrylat, Ethylenglykoldiacrylat, Ethylenglykoldimethacrylat, Propylenglykoldiacrylat, Propylenglykoldimethacrylat, Butandioldiacrylat, Butandioldimethacrylat, Hexandioldiacrylat, Hexandioldimethacrylat, Diethylenglykoldiacrylat, Diethylenglykoldimethacrylat, Triethylenglykoldiacrylat, Triethylenglykoldimethacrylat, Dipropylenglykoldiacrylat, Dipropylenglykoldimethacrylat, Tripropylenglykoldiacrylat, Tripropylenglykoldimethacrylat, Allylmethacrylat, Diacrylate und Dimethacrylate von Blockcopolymerisaten aus Ethylenoxid und Propylenoxid, zwei-, drei-, vier- oder fünffach mit Acrylsäure oder Methacrylsäure veresterte mehrwertige Alkohole, wie Glycerin, Trimethylolpropan, Pentaerythrit oder Dipentaerythrit, Ester monoethylenisch ungesättigter Carbonsäuren mit ethylenisch ungesättigten Alkoholen wie Allylalkohol, Cyclohexenol und Dicyclopentenylalkohol, z. B. Allylacrylat und Allylmethacrylat, weiterhin Triallylamin, Dialkyldiallylammoniumhalogenide wie Dimethyldiallylammoniumchlorid und Diethyldiallylammoniumchlorid, Tetraallylethylendiamin, Divinylbenzol, Diallylphthalat, Polyethylenglykoldivinylether von Polyethylenglykolen eines Molekulargewichtes von 106 bis 4000, Trimethylolpropandiallylether, Butandioldivinylether, Pentaerythrittriallylether, Umsetzungsprodukte von 1 Mol Ethylenglykoldiglycidylether oder Polyethylenglykoldiglycidylether mit 2 Mol Pentaerythritoltriallylether oder Allylalkohol, und Divinylethylenharnstoff. Der Anteil der Monomere C1 an der zu polymerisierenden Monomermischung beträgt vorzugsweise 0,01 bis 5.Gew.-% und insbesondere 0,2 bis 3 Gew.-%.

Als vernetzend wirkende Verbindungen C können ferner polyfunktionelle Verbindungen C2 fungieren, die wenigstens zwei z. B. 2, 3, 4 oder 5 funktionelle Gruppen aufweisen, die hinsichtlich ihrer Reaktivität gegenüber der Carboxylgruppe des Polymers komplementär sind. Als Vernetzer C kommen auch vernetzend wirkende Monomere C3 in Betracht, die neben einer ethylenisch ungesättigten Doppelbindung wenigstens eine weitere gegenüber Carboxylgruppen komplementäre funktionelle Gruppe aufweisen. In Betracht kommen auch Polymere mit einer Vielzahl derartiger funktioneller Gruppen. Geeignete funktionelle Gruppen sind z. B. Hydroxyl-, Amino-, Epoxy- und Aziridingruppen, weiterhin Isocyanat-, Ester- und Amidogruppen sowie Alkyloxysilylgruppen. Zu den geeigneten Vernetzern dieses Typs zählen beispielsweise Aminoalkohole, wie Ethanolamin oder Triethanolamin, Di- und Polyole, wie 1,3-Butandiol, 1,4-Butandiol, Ethylenglykol, Diethylenglykol, Triethylenglykol, Tetraethylenglykol, Polyethylenglykol, Glycerin, Polyglycerin, Propylenglykol, Polypropylenglykol, Trimethylolpropan, Pentaerythrit, Polyvinylalkohol, Sorbit, Stärke, Blockcopolymerisate aus Ethylenoxid und Propylenoxid, Polyamine wie Ethylendiamin, Diethylentriamin, Triethylentetramin, Tetraethylenpentamin, Pentaethylenhexamin und Polyethylenimine sowie Polyamine mit Molmassen von jeweils bis zu 4000000, Ester wie Sorbitanfettsäureester, ethoxylierte Sorbitanfettsäureester, Polyglycidylether wie Ethylenglykoldiglycidylether, Polyethylenglykol-diglycidylether, Glycerindiglycidylether, Glycerinpolyglycidylether, Diglycerinpolyglycidylether, Polyglycerinpolyglycidylether, Sorbitpolyglycidylether, Pentaerythritpolyglycidylether, Propylenglykoldiglycidylether und Polypropylenglykoldiglycidylether, Polyaziridinverbindungen wie 2,2-Bishydroxymethylbutanol-tris[3-(1-aziridinyl)-propionat], Diamide der Kohlensäure, wie 1,6-Hexamethylendiethylenharnstoff, Diphenylmethan-bis-4,4'-N,N'-diethylenharnstoff, Halogenepoxyverbindungen, wie Epichlorhydrin und a-Methylepifluorhydrin, Polyisocyanate, wie 2,4-Toluylendiisocyanat und Hexamethylendiisocyanat, Alkylencarbonate wie 1,3-Dioxolan-2-on und 4-Methyl-1,3-dioxolan-2-on, weiterhin Bisoxazoline und Oxazolidone, Polyamidoamine sowie deren Umsetzungsprodukte mit Epichlorhydrin, ferner polyquaternäre Amine, wie Kondensationsprodukte von Dimethylamin mit Epichlorhydrin, Homo- und Copolymere von Diallyldimethylammoniumchlorid sowie Homo- und Copolymerisate von Dimethylaminoethyl(meth)acrylat, die gegebenenfalls mit beispielsweise Methylchlorid quaterniert sind. Beispiele für Verbindungen C3 sind Hydroxyalkylacrylate und -methacrylate sowie Glycidylester der vorgenannten-ethylenisch ungesättigten Carbonsäuren und ethylenisch ungesättigte Glycidylether.

Vorzugsweise umfassen die Monomere C wenigstens ein Monomer C1 in den obengenannten Mengen. Vorzugsweise erfolgt die Polymerisation in Abwesenheit von Verbindungen C2.

Geeignete Pfropfgrundlagen können natürlichen oder synthetischen Ursprungs sein. Hierzu zählen Stärken, d. h. native Stärken aus der Gruppe der Maisstärke, Kartoffelstärke, Weizenstärke, Reisstärke, Tapiokastärke, Sorghumstärke, Maniokstärke, Erbsenstärke oder deren Mischungen, modifizierte Stärken, Stärkeabbauprodukte, z. B. oxidativ, enzymatisch oder hydrolytisch abgebaute Stärken, Dextrine, z. B. Röstdextrine sowie niedere Oligo- und Polysaccharide, z. B. Cyclodextrine mit 4 bis 8 Ringgliedern. Als Oligo- und Polysaccharide kommen weiterhin Cellulose, Stärke- und Cellulosederivate in Betracht. Ferner eignen sich Polyvinylalkohole, Homo- und Copolymere des N-Vinylpyrrolidons, Polyamine, Polyamide, hydrophile Polyester oder Polyalkylenoxide, insbesondere Polyethylenoxid und Polypropylenoxid. Geeignete Polyalkylenoxide weisen die allgemeine Formel I auf, 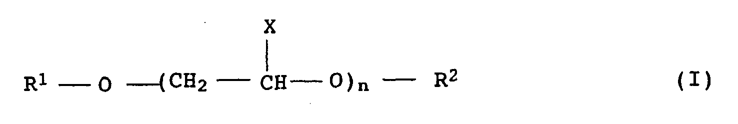 worin R¹, R² unabhängig voneinander für Wasserstoff; C₁-C₄-Alkyl; C₂-C₆-Alkenyl; Aryl, insbesondere Phenyl; oder (Meth)acryl stehen; X für Wasserstoff oder Methyl und n für eine ganze Zahl von 1 bis 1000, insbesondere 10 bis 400 stehen.

Als Polymerisationsreaktoren kommen die zur Herstellung üblichen Reaktoren, im Falle der Lösungspolymerisation insbesondere Bandreaktoren, Extruder und Kneter, in Betracht (siehe "Modern Superabsorbent Polymer Technology", Kapitel 3.2.3). Die Polymerisate werden besonders bevorzugt nach einem kontinuierlichen oder diskontinuierlichen Knetverfahren hergestellt.

Als Initiatoren kommen grundsätzlich alle Verbindungen in Betracht, die beim Erwärmen auf Polymerisationstemperatur unter Bildung von Radikalen zerfallen. Die Polymerisation kann auch durch Einwirkung energiereicher Strahlung, z. B. UV-Strahlung, in Gegenwart von Photoinitiatoren ausgelöst werden. Auch eine Initiierung der Polymerisation durch Einwirkung von Elektronenstrahlen auf die polymerisierbare, wässrige Mischung ist möglich.

Geeignete Initiatoren sind beispielsweise Peroxoverbindungen wie organische Peroxide, organische Hydroperoxide, Wasserstoffperoxid, Persulfate, Perborate, Azoverbindungen und die sogenannten Redoxkatalysatoren. Bevorzugt werden wasserlösliche Initiatoren. In manchen Fällen ist es vorteilhaft, Mischungen verschiedener Polymerisationsinitiatoren zu verwenden, z. B. Mischungen aus Wasserstoffperoxid und Natrium- oder Kaliumperoxodisulfat. Geeignete organische Peroxide sind beispielsweise Acetylacetonperoxid, Methylethylketonperoxid, tert-Butylhydroperoxid, Cumolhydroperoxid, tert-Amylperpivalat, tert-Butylperpivalat, tert-Butylperneohexanoat, tert-Butylperisobutyrat, tert-Butyl-per-2-ethylhexanoat, tert-Butylperisononanoat, tert-Butylpermaleat, tert-Butylperbenzoat, Di-(2-ethylhexyl)peroxidicarbonat, Dicyclohexylperoxidicarbonat, Di-(4-tert.-butylcyclohexyl)peroxidicarbonat, Dimyristilperoxidicarbonat, Diacetylperoxi-dicarbonat, Allylperester, Cumylperoxyneodecanoat, tert.-Butylper-3,5,5-trimethylhexanoat, Acetylcyclohexylsulfonylperoxid, Dilaurylperoxid, Dibenzoylperoxid und tert.-Amylperneodekanoat. Besonders geeignete Polymerisationsinitiatoren sind wasserlösliche Azostarter, z. B. 2,2'-Azo-bis-(2-amidinopropan)dihydrochlorid, 2,2'-Azo-bis-(N,N'-dimethylen)isobutyramidin-dihydrochlorid, 2-(Carbamoylazo)isobutyronitril, 2,2'-Azobis[2-(2'-imidazolin-2-yl)propan]dihydrochlorid und 4,4'-Azobis-(4-cyanovaleriansäure). Die genannten Polymerisationsinitiatoren werden in üblichen Mengen eingesetzt, z. B. in Mengen von 0,01 bis 5 Gew.-%, vorzugsweise 0,05 bis 2,0 Gew.-%, bezogen auf die zu polymerisierenden Monomeren.

Die bevorzugten Redoxinitiatoren zählen zu den wasserlöslichen Initiatoren und enthalten als oxidierende Komponente mindestens eine der oben angegebenen Peroxoverbindungen und als reduzierende Komponente beispielsweise Ascorbinsäure, Glukose, Sorbose, Ammonium- oder Alkalimetallsulfit, -hydrogensulfit, -thiosulfat, -hyposulfit, -pyrosulfit oder -sulfid, Metallsalze, wie Eisen(II)-ionen oder Natriumhydroxymethylsulfoxylat. Vorzugsweise verwendet man als reduzierende Komponente des Redoxkatalysators Ascorbinsäure oder Natriumsulfit. Bezogen auf die bei der Polymerisation eingesetzte Menge an Monomeren verwendet man beispielsweise 3 x 10⁻⁶ bis 1 Mol-% der reduzierenden Komponente des Redoxkatalysatorsystems und 0,001 bis 5,0 Mol-% der oxidierenden Komponente des Redoxkatalysators.

Wenn man die Polymerisation durch Einwirkung energiereicher Strahlung auslöst, verwendet man üblicherweise als Initiator sogenannte Photoinitiatoren.

Die Herstellung des Hydrogels in Schritt a) des erfindungsgemäßen Verfahrens kann auch eine nachträgliche Vernetzung des Gels umfassen. Bei der nachträglichen Vernetzung (sog. Gelvernetzung) werden Polymere, die durch die Polymerisation von Acrylsäure und gegebenenfalls monoethylenisch ungesättigten Comonomeren B und/oder C, insbesondere C1, hergestellt wurden, mit Verbindungen C2 umgesetzt, die mindestens zwei gegenüber den Carboxylgruppen reaktive Gruppen aufweisen. Diese Umsetzung kann bei Raumtemperatur oder aber bei erhöhten Temperaturen bis zu 220 °C erfolgen. Zur nachträglichen Vernetzung (Gelvernetzung) werden die Vernetzer C2 den erhaltenen Polymeren in Mengen von 0,5 bis 20 Gew.-%, bevorzugt von 1 bis 14 Gew.-%, bezogen auf die Menge des Polymers, zugesetzt.

Die in Schritt a) erhaltenen Polymerisate fallen in der Regel als Hydrogele an. Ihr Feuchtigkeitsgehalt liegt in der Regel im Bereich 20 bis 80 Gew.-%. Das so erhaltene Hydrogel wird dann in an sich bekannter Weise in ein teilchenförmiges Hydrogel oder ein Hydrogel bildendes-Pulver überführt.

Hierzu wird das bei der Polymerisation anfallende Hydrogel in der Regel zunächst nach bekannten Methoden zerkleinert. Die Grobzerkleinerung der Hydrogele erfolgt mittels üblicher Reiss- und/oder Schneidwerkzeuge, z. B. durch die Wirkung einer Austragspumpe im Falle der Polymerisation in einem zylindrischen Reaktor oder durch eine Schneidwalze oder Schneidwalzenkombination im Falle der Bandpolymerisation.

Sofern die Monomermischung in nicht neutralisierter Form eingesetzt wurde, kann man das erhaltene saure Polymerisat in an sich bekannter Weise auf den gewünschten Neutralisationsgrad von in der Regel wenigstens 25 mol-%, vorzugsweise wenigstens 50 mol-%, insbesondere 90 bis 100 mol-%, bezogen auf Säuregruppen tragende Monomereinheiten, bringen. Alternativ kann die Einstellung des Neutralisationsgrades auch während der Polymerisation, z. B. im Kneter, vorgenommen werden.

Das so erhaltene, vorzugsweise neutralisierte oder teilweise neutralisierte Polymerisat wird anschließend bei erhöhter Temperatur, z. B. im Bereich von 80 °C bis 250 °C und insbesondere im Bereich von 100 °c bis 180 °C, nach bekannten Verfahren getrocknet (siehe "Modern Superabsorbent Polymer Technology" Kapitel 3.2.5). Hierbei erhält man die Polymerisate in Form von Pulvern oder Granulaten, die gegebenenfalls zur Einstellung der Partikelgröße noch mehreren Mahl- und Siebvorgängen unterworfen werden (siehe "Modern Superabsorbent Polymer Technology" Kapitel 3.2.6 und 3.2.7).

Vorzugsweise umfasst das erfindungsgemäße Verfahren eine Oberflächennachvernetzung. Die Oberflächennachvernetzung erfolgt in an sich bekannter Weise mit getrockneten, vorzugsweise gemahlenen und abgesiebten Polymerpartikeln oder mit Hydrogelen. Zur Oberflächenvernetzung werden Verbindungen eingesetzt, die wenigstens zwei funktionelle Gruppen aufweisen, die mit den funktionellen Gruppen, vorzugsweise den Carboxylgruppen der in Schritt a) erhaltenen Polymere unter Vernetzung reagieren können (Nachvernetzungsmittel). Die funktionellen Gruppen können im Nachvernetzungsmittel in latenter Form vorliegen, d.h. sie werden erst unter den Reaktionsbedingungen der Oberflächennachvernetzung freigesetzt. Geeignete funktionelle Gruppen in Nachvernetzungsmitteln sind Hydroxylgruppen, Glycidylgruppen, Alkoxysilylgruppen, Aziridingruppen, primäre und sekundäre Aminogruppen, N-Methylolgruppen (= N-Hydroxymethylgruppen, N-CH₂-OH-Gruppen), Oxazolidin-Gruppen, Harnstoff- und Thioharnstoffgruppen, gegebenenfalls reversibel blockierte Isocycanat-Gruppen sowie cyclische Carbonat-Gruppen wie in Ethylencarbonat. Zur Oberflächennachvernetzung werden die Nachvernetzungsmittel, vorzugsweise in Form einer wässrigen Lösung auf die Oberfläche der Polymerisat-Partikel aufgebracht. Die wässrige Lösung kann wassermischbare organische Lösungsmittel enthalten. Geeignete Lösungsmittel sind z. B. C₁-C₄-Alkohole wie Methanol, Ethanol, Isopropanol oder Ketone wie Aceton und Methylethylketon.

Geeignete Nachvernetzungsmittel sind beispielsweise:
- Di- oder Polyglycidylverbindungen wie Phosphorsäuretriglycidylester und Phosphonsäurediglycidylester oder Ethylenglykoldiglycidylether sowie Bischlorhydrinether von Polyalkylenglykolen,
- Alkoxysilylverbindungen,
- Polyaziridine, Aziridin-Einheiten enthaltende Verbindungen auf Basis von Polyethern oder substituierten Kohlenwasserstoffen, beispielsweise Bis-N-aziridinomethan,
- Polyamine oder Polyamidoamine sowie deren Umsetzungsprodukte mit Epichlorhydrin,
- Diole und Polyole, z. B. Ethylenglykol, 1,2-Propandiol, 1,4-Butandiol, Glycerin, Methyltriglykol, Trimethylolethan, Trimethylolpropan, Polyethylenglykole mit einem mittleren Molekulargewicht M_{w} von 200 bis 10000, Di- und Polyglycerin, Pentaerythrit, Sorbit, die Oxethylate dieser Polyole sowie deren Ester mit Carbonsäuren oder mit Kohlensäure wie Ethylencarbonat oder Propylencarbonat,
- Kohlensäurederivate wie Harnstoff, Thioharnstoff, Guanidin, Dicyandiamid, 2-Oxazolidinon und dessen Derivate, Bisoxazolin, Polyoxazoline, Di- und Polyisocyanate,
- Di- und Poly-N-methylolverbindungen wie beispielsweise Methylenbis(N-methylol-methacrylamid) oder Melamin-FormaldehydHarze,
- Verbindungen mit zwei oder mehr blockierten Isocyanat-Gruppen wie beispielsweise Trimethylhexamethylendiisocyanat blockiert mit 2,2,3,6-Tetramethyl-piperidinon-4.

In einer besondere Ausführungsform des erfindungsgemäßen Verfahrens werden solche Vernetzungsmittel eingesetzt, die mit den Carboxylgruppen des Polymerisats Estergruppen bilden. Beispiele hierfür sind die vorgenannten Diole und Polyole, deren Ester mit Carbonsäuren oder mit Kohlensäure sowie Di- und Polyglycidylverbindungen und deren Mischungen.

Bei Bedarf können saure Katalysatoren wie p-Toluolsulfonsäure, Phosphorsäure, Borsäure oder Ammoniumdihydrogenphosphat zugesetzt werden.

Das Aufbringen der Vernetzer-Lösung erfolgt bevorzugt durch Aufsprühen einer Lösung des Vernetzers in herkömmlichen Reaktionsmischern oder Misch- und Trocknungsanlagen wie beispielsweise Patterson-Kelly-Mischer, DRAIS-Turbulenzmischer, Lödige-Mischer, Schneckenmischer, Tellermischer, Wirbelschichtmischer und Schugi-Mix. Nach Aufsprühen der Vernetzer-Lösung kann ein Temperaturbehandlungsschritt nachfolgen, bevorzugt in einem nachgeschalteten Trockner, bei einer Temperatur zwischen 80 und 230 °C, bevorzugt 80 bis 190 °C, und besonders bevorzugt zwischen 100 und 160 °C, über einen Zeitraum von 5 Minuten bis 6 Stunden, bevorzugt 10 Minuten bis 2 Stunden und besonders bevorzugt 10 Minuten bis 1 Stunde, wobei sowohl Spaltprodukte als auch Lösungsmittelanteile entfernt werden können. Die Trocknung kann aber auch im Mischer selbst erfolgen, durch Beheizung des Mantels oder Einblasen eines vorgewärmten Trägergases.

Die nach dem erfindungsgemäßen Verfahren erhaltenen SAP auf Basis von Acrylsäure zeichnen sich durch einen geringen Restmonomergehalt aus und sind zudem in der Regel besonderes geruchsneutral, d.h. sie weisen anders als die bislang bekannten SAP nurmehr einen sehr schwachen oder keinen unangenehmen Geruch mehr auf. Sie sind daher insbesondere zur Herstellung von Hygieneartikeln geeignet.

Die vorliegende Erfindung betrifft somit auch die nach diesem Verfahren erhältlichen SAP und deren Verwendung zur Herstellung von Hygieneartikeln wie Windeln, Inkontinenzeinlagen und -hosen, Tampons oder Damenbinden. Weiterhin betrifft die vorliegende Erfindung Hygieneartikel mit einem Absorptionskörper, der wenigstens ein erfindungsgemäßes wasserabsorbierendes Mittel enthält.

Der Aufbau und die Form von Hygieneartikeln, insbesondere Windeln, Binden und Inkontinenzeinlagen und -hosen für Erwachsene, ist allgemein bekannt und beispielsweise in der EP-A-0 316 518, der EP-A-0 202 127, der DE 19737434, der WO 00/65084, insbesondere S. 6-15, der WO 00/65348, insbesondere S. 4-17 und der WO 00/35502, insbesondere S. 3-9 beschrieben.

Typische Hygieneartikel in Form von Windeln, Binden und Inkontinenzeinlagen und -hosen umfassen:
(A) eine obere flüssigkeitsdurchlässige Abdeckung
(B) eine untere flüssigkeitsundurchlässige Schicht
(C) einen zwischen (A) und (B) befindlichen Kern, enthaltend
   (C1) 10 - 100 Gew.-% des erfindungsgemäßen SAP
   (C2)0 - 90 Gew.-% hydrophiles Fasermaterial
(D) gegebenenfalls eine sich unmittelbar oberhalb und unterhalb des Kerns (C) sich befindende Tissueschicht und
(E) gegebenenfalls eine zwischen (A) und (C) sich befindende Aufnahmeschicht.

Bei der flüssigkeitsdurchlässigen Abdeckung (A) handelt es sich um die Schicht, die direkten Hautkontakt hat. Das Material hierfür besteht hierbei aus üblichen synthetischen oder halbsynthetischen Fasern oder Filmen von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen.

Die flüssigkeitsundurchlässige Schicht (B) besteht in der Regel aus einer Folie beispielsweise aus Polyethylen oder Polypropylen.

Der Kern (C) enthält neben dem erfindungsgemäßen Hydrogel-formendem Pfropfpolymer (C1) hydrophiles Fasermaterial (C2). Unter hydrophil ist zu verstehen, dass sich wässrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 bis 200 µm, bevorzugt 10 bis 100 µm. Darüber hinaus haben die Fasern eine Mindestlänge von 2 mm.

Der Anteil des hydrophilen Fasermaterials bezogen auf die Gesamtmenge des Kerns beträgt bevorzugt 20 bis 80 Gew.-%, besonders bevorzugt 40 bis 70 Gew.-%.

Die nach dem erfindungsgemäßen Verfahren hergestellten Superabsorber sowie die unter deren Verwendung hergestellten Hygieneartikel zeichnen sich überraschenderweise durch einen besonders geringen Restmonomergehalt von in der Regel ≤ 100 ppm, und insbesondere ≤ 50 ppm aus, bezogen auf das Trockengewicht des Hydrogel bildenden Polymerisats. Zudem weisen die Superabsorber einen geringen Eigengeruch auf.

Die folgenden Beispiele sollen die Erfindung verdeutlichen, ohne jedoch einschränkend zu sein.

In allen Fällen wurde eine Reinacrylsäure eingesetzt, die aus einer nach dem Verfahren der DE-A 4308087 hergestellten Rohacrylsäure durch Kristallisation oder Destillation gewonnen wurde, wobei in allen Fällen der Gehalt an aromatischen Aldehyden < 5 ppm und der Gehalt an Prozessinhibitoren < 5 ppm war. Der Gehalt an Diacrylsäure und höheren Oligomeren der Acrylsäure sowie der Gehalt an aliphatischen Carbonsäuren wurde durch Aufstocken der Reinacrylsäuren mit den jeweiligen Verunreinigungen eingestellt. Die Konzentration an Verunreinigungen ist in Tabelle 1 angegeben.

### Allgemeine Herstellungsvorschrift:

Aus 1735 g einer Reinacrylsäure A, 1445 g einer 50 gew.-%igen wässrigen Natronlauge und 2760 g Wasser wurde eine teilneutralisierte Acrylsäure/Natriumacrylat-Lösung hergestellt und in üblicher Weise durch Behandeln mit Stickstoff in einer Strippkolonne im Gegenstrom von Sauerstoff befreit. Die weitgehend sauerstofffreie Lösung wurde in einen Trogkneter mit Mantelheizung (Typ LUK 8 der Fa. Werner und Pfleiderer) überführt und unter Durchmischung mit 7,8 g Polyethylenglykoldiacrylat versetzt. Der Reaktor wurde während der gesamten Reaktionsdauer mit Stickstoff überlagert. Bei eingeschalteten Rührwellen gab man zunächst 33,12 g Natriumperoxodisulfat als 15 gew.-%ige wässrige Lösung und anschließend 20,79 g Ascorbinsäure als 0,5 gew.-%ige Lösung zu. Nach Beendigung der Zugabe wurde der Inhalt des Kneters erhitzt (Temperatur der Heizflüssigkeit 74°C). Hierbei kam es zu einer spontanen Erwärmung des Kneterinhaltes und einer starken Zunahme der Viskosität. Sobald die maximale Temperatur des Kneterinhaltes überschritten war, stellte man die Heizung ab und ließ 15 min. nachpolymerisieren. Man kühlte den Inhalt des Kneters auf 50 bis 60°C ab, gab ihn in dünner Schicht auf ein Trocknungssieb und trocknete ihn 90 min. bei 160 °C. Anschließend zerkleinerte man das getrocknete Polymerisat durch Mahlen und Sieben auf eine Endkorngröße von 100 bis 850 µm.

1,8 kg des so hergestellten Pulvers wurden in einem Lödige Pflugscharmischer mit 5 l Inhalt vorgelegt. Hierauf sprühte man innerhalb von 5 bis 10 min. eine Lösung von 1,4 g Ethylenglykoldiglycidylether in 59 g Wasser und 29 g 1,2-Propandiol. Man erwärmte auf 120°C und behielt die Temperatur 60 min. bei, wobei das Lösungsmittel abdestillierte. Anschließend kühlte man ab und siebte die Kornfraktion 100 bis 850 µm ab.

Das so erhaltene Produkt wurde hinsichtlich seiner Restmonomere (Acrylsäure und β-Hydroxypropionsäure) nach der folgenden Methode untersucht. 1 g des nach der oben angegebenen Vorschrift erhaltenen Polymers wurde 3 Stunden in entmineralisiertem Wasser gerührt und die Suspension anschließend filtriert. Mittels chromatographischer Analyse (HPLC) des wässrigen Filtrats wurden der Gehalt an Restmonomere (Acrylsäure) bestimmt. Die gefundenen Werte sind in Tabelle 1 angegeben:

Die so hergestellten Superabsorber wurden außerdem einem Geruchstest unterworfen. Hierzu wurden jeweils 5 Proben ä 15 g des jeweiligen Superabsorbers in gasdicht verschlossenen Probengefäßen 5 h bei 30 °C getempert. Anschließend wurde von 5 Testpersonen der Geruch der Probe nach einer Notenskala von 1 bis 5 bewertet, wobei 1 für keinen oder einen kaum wahrnehmbaren Eigengeruch, 2 für einen geringen, 3 für einen deutlichen, 4 für einen starken und 5 für einen sehr starken Eigengeruch steht. Die Mittelwerte der individuellen Geruchsergebnisse sind für die jeweiligen SAP in Tabelle 1 dargestellt.

**Tabelle 1:**

| | Diacrylsäure [ppm] | höhere Oli gomere¹ [ppm] | aliphatische Carbonsäuren [ppm] | Restmono mergehalt [ppm] | Geruch |
|---|---|---|---|---|---|
| 1 | 150 | <10 | 180 | <30 | 1,2 |
| 2 | 300 | <10 | 250 | <30 | 1,4 |
| 3 | 150 | <10 | 1500 | <30 | 4,0 |
| 4 | 300 | <10 | 1500 | <30 | 4,0 |
| 5 | 300 | <10 | 700 | <50 | 3,4 |
| 6 | 320 | 150 | 250 | 95 | 1,6 |
| V1 | 350 | 500 | 250 | 150 | 2,0 |
| V2 | 2000 | 100 | 1500 | 220 | 4,6 |

| | | | | | |
|---|---|---|---|---|---|
| ¹) Triacrylsäure und höhere Oligomere (Verbindungen I mit X ≥ 2) | | | | | |

## Patentansprüche

1. Verfahren zur Herstellung eines geruchsarmen, Hydrogel-bildenden Polymerisats auf Basis von Acrylsäure, umfassend die folgenden Schritte:
a) Herstellung eines polymeren Hydrogels durch radikalische Polymerisation einer wenigstens 50 Gew.-% Acrylsäure enthaltenden Monomerzusammensetzung in einem wässrigen Polymerisationsmedium und Überführen des Hydrogels in ein teilchenförmiges Hydrogel oder ein Hydrogel bildendes Pulver; und gegebenenfalls
b) Behandeln des teilchenförmigen Hydrogels oder des Hydrogel bildenden Pulvers mit einer vernetzend wirkenden Substanz, die wenigstens zwei gegenüber den Carboxylgruppen des Polymerisats reaktive funktionelle Gruppen, gegebenenfalls_in latenter Form, aufweist;
**dadurch gekennzeichnet, dass** die in Schritt a) eingesetzte Acrylsäure weniger als 500 ppm (Gewichtsanteile bezogen auf Acrylsäure) Oligomere der Acrylsäure enthält.

2. Verfahren nach Anspruch 1, **dadurch gekennzeichnet, dass** man in Schritt a) eine Acrylsäure einsetzt, deren Gesamtgehalt an Triacrylsäure und höheren Oligomeren der Acrylsäure weniger als 100 ppm und speziell weniger als 50 ppm beträgt.

3. Verfahren nach Anspruch 1 oder 2, **dadurch gekennzeichnet, dass** man in Schritt a) eine Acrylsäure einsetzt, die durch eine ein- oder mehrstufige Kristallisation einer Roh-Acrylsäure mit einem Gehalt an Oligomeren der Acrylsäure im Bereich von 0,07 bis 3 Gew.-% gewonnen wurde.

4. Verfahren nach Anspruch 3, **dadurch gekennzeichnet, dass** man in Schritt a) eine Acrylsäure einsetzt, die durch eine ein- oder mehrstufige Kristallisation der Rohacrylsäure bei einer Temperatur im Bereich von 0 bis 13 °C erhalten wurde.

5. Verfahren nach einem der vorhergehenden Ansprüche, **dadurch gekennzeichnet, dass** man in Schritt a) eine Acrylsäure einsetzt, die weniger als 500 ppm aliphatische Carbonsäuren, bezogen auf das Gewicht der Acrylsäure, enthält.

6. Verfahren nach einem der vorhergehenden Ansprüche, **dadurch gekennzeichnet, dass** man die Acrylsäure in Schritt a) in Form einer teilweise oder vollständig neutralisierten, wässrigen Acrylsäure-Lösung einsetzt.

7. Verfahren nach einem der vorhergehenden Ansprüche, **dadurch gekennzeichnet, dass** das Vernetzungsmittel in Schritt c) ausgewählt ist aus Verbindungen, die mit den Carboxylgruppen des Polymerisats Estergruppen bilden.

8. Verfahren nach einem der vorhergehenden Ansprüche, **dadurch gekennzeichnet, dass** die in Schritt a) zu polymerisierende Monomermischung, bezogen auf ihr Gesamtgewicht
- 50 bis 99,99 Gew.-% Acrylsäure als Monomer A,
- 0 bis 49,99 Gew.-% eines oder mehrerer mit Acrylsäure copolymerisierbarer, monoethylenisch ungesättigter Monomere B und
- 0,01 bis 30 Gew.-% wenigstens einer vernetzend wirkenden Verbindung C umfasst.

9. Verfahren nach einem der vorhergehenden Ansprüche, **dadurch gekennzeichnet, dass** man eine Acrylsäure mit einem Gehalt von Oligomeren der Acrylsäure < 500 ppm unmittelbar vor ihrer Verwendung in Schritt a) bereitstellt.

10. Verwendung einer Acrylsäure mit einem Gehalt an Oligomere der Acrylsäure von weniger als 500 ppm zur Herstellung eines geruchsarmen, Hydrogel-bildenden Polymerisats auf Basis von Acrylsäure.

## Claims

1. A process for preparing a low-odor hydrogel-forming acrylic acid polymer, which comprises the steps of:
a) preparing a polymeric hydrogel by free-radically polymerizing a monomer composition comprising at least 50% by weight of acrylic acid in an aqueous polymerization medium and converting said hydrogel into a particulate hydrogel or into hydrogel-forming powder; and if appropriate
b) treating said particulate hydrogel or said hydrogel-forming powder with a crosslinking substance which, actually or latently, comprises at least two functional groups capable of reacting with the carboxyl groups on the polymer;
**characterized by** the acrylic acid used in step a) comprising less than 500 ppm (by weight, based on acrylic acid) of acrylic acid oligomers.

2. The process according to claim 1 wherein step a) is effected using an acrylic acid whose total level of triacrylic acid and higher oligomers of acrylic acid is less than 100 ppm and specifically less than 50 ppm.

3. The process according to claim 1 or 2 wherein step a) is effected using an acrylic acid obtained by a single or multiple stage crystallization of a crude acrylic acid having a content of oligomers of the acrylic acid in the range from 0.07% to 3% by weight.

4. The process according to claim 3 wherein step a) is effected using an acrylic acid obtained by single or multiple stage crystallization of said crude acrylic acid at from 0 to 13°C.

5. The process according to any preceding claim wherein the acrylic acid used in step a) comprises less than 500 ppm of aliphatic carboxylic acids, based on the weight of the acrylic acid.

6. The process according to any preceding claim wherein the acrylic acid used in step a) is in the form of a partially or completely neutralized aqueous acrylic acid solution.

7. The process according to any preceding claim wherein the crosslinker in step c) is selected from compounds capable of forming ester groups with the carboxyl groups on the polymer.

8. The process according to any preceding claim wherein the monomer mixture to be polymerized in step a) comprises, based on its total weight,
- from 50% to 99.99% by weight of acrylic acid as monomer A,
- from 0% to 49.99% by weight of one or more monoethylenically unsaturated monomers B which are copolymerizable with acrylic acid, and
- from 0.01% to 30% by weight of at least one crosslinking compound C.

9. The process according to any preceding claim wherein an acrylic acid having an acrylic acid oligomer content < 500 ppm is provided directly prior to its use in step a).

10. The use of an acrylic acid having an acrylic acid oligomer content of less than 500 ppm for preparing a low-odor hydrogel-forming acrylic acid polymer.

## Revendications

1. Procédé de préparation d'un polymère formateur d'hydrogel peu odorant à base d'acide acrylique, comprenant les étapes suivantes :
a) une préparation d'un hydrogel polymère par polymérisation radicalaire d'une composition monomère contenant au moins 50 % en poids d'acide acrylique dans un milieu de polymérisation aqueux et une conversion de l'hydrogel en un hydrogel particulaire ou en une poudre formatrice d'hydrogel, et éventuellement
b) un traitement de l'hydrogel particulaire ou de la poudre formatrice d'hydrogel par une substance à action de réticulation qui présente au moins deux groupes fonctionnels réactifs vis-à-vis des groupes carboxyle du polymère, éventuellement sous une forme latente,
**caractérisé en ce que** l'acide acrylique mis en oeuvre dans l'étape a) contient moins de 500 ppm (fractions en poids par rapport à l'acide acrylique) d'oligomères de l'acide acrylique.

2. Procédé suivant la revendication 1, **caractérisé en ce qu'**on met en oeuvre dans l'étape a) un acide acrylique dont la teneur totale en acide triacrylique et en oligomères supérieurs de l'acide acrylique est inférieure à 100 ppm et spécialement à 50 ppm.

3. Procédé suivant la revendication 1 ou 2, **caractérisé en ce qu'**on met en oeuvre dans l'étape a) un acide acrylique qui est obtenu par une cristallisation en une ou plusieurs étapes d'un acide acrylique brut présentant une teneur en oligomères de l'acide acrylique de l'ordre de 0,07 à 3 % en poids.

4. Procédé suivant la revendication 3, **caractérisé en ce qu'**on met en oeuvre dans l'étape a) un acide acrylique qui a été obtenu par cristallisation en une ou plusieurs étapes de l'acide acrylique brut à une température de l'ordre de 0 à 13°C.

5. Procédé suivant l'une des revendications précédentes, **caractérisé en ce qu'**on met en oeuvre dans l'étape a) un acide acrylique qui contient moins de 500 ppm d'acides carboxyliques aliphatiques, par rapport au poids de l'acide acrylique.

6. Procédé suivant l'une des revendications précédentes, **caractérisé en ce qu'**on met en oeuvre l'acide acrylique dans l'étape a) sous la forme d'une solution aqueuse d'acide acrylique, partiellement ou totalement neutralisée.

7. Procédé suivant l'une des revendications précédentes, **caractérisé en ce que** l'agent de réticulation de l'étape c) est choisi parmi des composés qui forment des groupes ester avec les groupes carboxyle du polymère.

8. Procédé suivant l'une des revendications précédentes, **caractérisé en ce que** le mélange monomère à polymériser dans l'étape a) comporte, par rapport à son poids total,
- 50 à 99,99 % en poids d'acide acrylique comme monomère A,
- 0 à 49,99 % en poids d'un ou de plusieurs monomères monoéthyléniquement insaturés B, copolymérisables avec de l'acide acrylique, et
- 0,01 à 30 % en poids d'au moins un composé à action de réticulation C.

9. Procédé suivant l'une des revendications précédentes, **caractérisé en ce qu'**on prépare un acide acrylique ayant une teneur en oligomères de l'acide acrylique < 500 ppm immédiatement avant son utilisation dans l'étape a).

10. Utilisation d'un acide acrylique ayant une teneur en oligomères de l'acide acrylique inférieure à 500 ppm, pour la préparation d'un polymère formateur d'hydrogel peu odorant à base d'acide acrylique.
